# EUROPÄISCHE PATENTSCHRIFT

(11) **EP 0 857 315 B1**
(45) Veröffentlichungstag und Bekanntmachung des Hinweises auf die Patenterteilung: **18.12.2002**
(21) Anmeldenummer: 96933258.4
(22) Anmeldetag: 16.10.1996
(51) Int. Cl.: G02B 21/08, A61B 3/13, A61B 3/15

(54) **ABBILDENDES OPTISCHES GERÄT**
IMAGING OPTICAL INSTRUMENT
APPAREIL D'IMAGERIE OPTIQUE

(30) Priorität: 23.10.1995 DE 19539371
(43) Veröffentlichungstag der Anmeldung: 12.08.1998
(73) Patentinhaber: STEINHUBER, Wolfdietrich, 6080 Igls (AT)
(72) Erfinder: STEINHUBER, Wolfdietrich, 6080 Igls (AT)
(74) Vertreter: Torggler, Paul Norbert, Dr.
(86) Internationale Anmeldenummer: AT9600197
(87) Internationale Veröffentlichungsnummer: WO97015855

(56) Entgegenhaltungen:
- EP-A- 0 608 516
- WO-A-83/02717
- DE-A- 3 151 837
- DE-A- 3 714 041
- DE-B- 1 223 581
- SCANNING, Bd. 12, XP000255795 BURNS D H ET AL: "SCANNING SLIT APERTURE CONFOCAL MICROSCOPY FOR THREE-DIMENSIONAL IMAGING"

## Beschreibung

Die Erfindung betrifft ein abbildendes optisches Gerät entsprechend dem Oberbegriff von Anspruch 1.

Eine Einrichtung dieser Art ist aus der US-PS 3,547,512 bekannt. Durch die spaltförmigen Blenden im Beobachtungsstrahlengang und im Beleuchtungsstrahlengang wird immer nur ein kleiner Bereich des Objektes beleuchtet und gleichzeitig beobachtet. Um dennoch einen größeren Teil des Objektes untersuchen zu können, werden die Beleuchtungsblende und die Beobachtungsblende synchron bewegt, wodurch ein Teil des Objektes abgetastet wird. Wird das Objekt mit einem Detektor, der eine gewisse Trägheit aufweist beobachtet, wie beispielsweise dem menschlichen Auge, und erfolgt die Bewegung der Spaltbilder der Blenden schnell genug über immer wieder den gleichen Teil des Objektes, dann verschmelzen die Einzelbilder zu einem Gesamtbild des abgetasteten Teiles des Objektes.

Dieses Verfahren soll insbesondere die Betrachtung eines Objektes durch ein trübes bzw. opakes Medium hindurch verbessern, indem es die Erzeugung und Beobachtung von Streulicht und die daraus resultierende Blendung vermindert, und kann daher beispielsweise in der indirekten Ophthalmoskopie zur Beobachtung der Netzhaut des Auges eingesetzt werden. Dabei wird mittels einer Ophthalmoskopierlinse von der Netzhaut ein reelles Zwischenbild außerhalb des Auges erzeugt, wobei die Beleuchtungseinrichtung und das vergrößemde optische Systemn auf die Ebene dieses Zwischenbildes fokusiert sind.

Allerdings ist mit den bisher beschriebenen Maßnahmen alleine noch kein zufriedenstellendes Ergebnis zu erreichen. In der US-PS 3,547,512 werden daher zusätzliche Blenden bzw. Spiegel verwendet, die jeweils die Hälfte des Beobachtungs- bzw. Beleuchtungsstrahlenbündels ausblenden, sodaß das Beobachtungsstrahlenbündel und das Beleuchtungsstrahlenbündel mit Ausnahme eines kleinen Schnittbereiches in der Objektebene getrennt voneinander verlaufen. Zwar wird dadurch das erzeugte bzw. beobachtete Streulicht erfolgreich weiter verringert, allerdings werden durch die Einschränkungen der Aperturen des Beobachtungsstrahlenbündels und des Beleuchtungsstrahlenbündels die Lichtstärke und die erreichbare Auflösung stark verringert. Ähnliche Einrichtungen mit den gleichen Nachteilen sind auch aus der US-PS 4,170,398 und der EP-A2 197 761 bekannt.

Eine andere Methode zur Verringerung der Blendung ist das Dunkelfeldverfahren, bei dem das Beleuchtungsstrahlenbündel nicht in das Objektiv abgebildet wird, wenn kein Objekt vorhanden ist. Erst die Beugung am Objekt führt zu beobachtbarem Licht, wobei aber kein naturgetreues Bild erzeugt wird, sodaß dieses Verfahren nur zur Konturendarstellung verwendet wird.

Aus der EP-A-0 608 516 ist ein abbildendes optisches Gerät zur indirekten Ophthalmoskopie bekannt, bei dem die optische Achse des Beobachtungsstrahlenbündels die optische Achse des Beleuchtungsstrahlenbündels in der Zwischenbildebene unter einem Winkel α schneidet. Die geneigte Einfallsrichtung des Lichts dient dabei dem Zweck, störende Reflexe zu vermeiden.

Aus der DE-A-37 14 041 ist ein abbildendes optisches Gerät bekannt, das ein Beleuchtungsstrahlenbündel mit einer spaltförmigen Blende sowie ein Beobachtungsstrahlenbündel mit einer Maske aufweist, wobei die Blende und die Maske sich synchron in Abhängigkeit zueinander bewegen. Allerdings verlaufen Beobachtungsstrahlenbündel und Beleuchtungsstrahlenbündel parallel zueinander in der Objektebene.

Aufgabe der Erfindung ist die Bereitstellung eines neuartigen vergrößernden oder verkleinernden optischen Gerätes, welches die bekannten Einrichtungen verbessert und auch bei einer Beobachtung durch trübe Medien ein Bild des Objektes erzeugt, das möglichst kontraststark und blendfrei ist, sodaß feinste Details sichtbar werden. Die Lichtstärke des Gerätes und die erreichbare Auflösung sollen dabei möglichst hoch sein.

Erfindungsgemäß gelingt dies bei einer Einrichtung entsprechend dem Oberbegriff von Anspruch 1 durch die kennzeichnenden Merkmale von Anspruch 1.

Ganz allgemein werden bei einem abbildenden optischen Instrument die von einem beleuchteten Objekt ausgehenden Lichtstrahlen durch ein Objektiv als Zwischenbild abgebildet, und dieses wird mittels eines Okulars vergrößert betrachtet. Dabei werden nach Abbe die beleuchtenden Lichtstrahlen vom Objekt gebeugt (moduliert), wobei Beugungen nullter, erster, usw. Ordnung auftreten und interferieren. Die lichtstarken Strahlen nullter Ordnung (ungebeugt) überlagern die lichtschwächeren Strahlen höherer Ordnung und machen diese schlechter sichtbar, weil nur gleichstarke Wellenfronten ein klares Interferenzbild erzeugen können.

Ein bedeutender Vorteil der Erfindung besteht nunmehr darin, daß durch das erfindungsgemäße Gerät die ungebeugten Strahlen nullter Ordnung zwar abgeschwächt werden, nicht aber ausgelöscht (wie beim Dunkelfeldverfahren), sodaß durch Angleichung der Amplituden von gebeugten und ungebeugten Strahlen der Kontrast bedeutend verbessert wird.

In einer bevorzugten Ausführungsform der Erfindung ist der Neigungswinkel zwischen Beobachtungsstrahlenbündel und Beleuchtungsstrahlenbündel einstellbar. Wenn die Spaltbreiten von Beobachtungsblende und Beleuchtungsblende dabei konstant gehalten werden, kann auf diese Weise der Kontrast eingestellt werden. Andererseits kann vorgesehen sein, den Neigungswinkel mit der Spaltbreite zu koppeln, wobei für eine größere Spaltbreite ein größerer Neigungswinkel eingestellt wird, um die Blendfreiheit bei allen einstellbaren Spaltbreiten bzw. Neigungswinkeln zu gewährleisten und den Kontrast konstant zu halten.

Eine Veränderung der Richtung des Beleuchtungsstrahlenbündels gegenüber der des Beobachtungsstrahlenbündels ist für einen Augenspiegel mit Beleuchtungseinrichtung aus der DE-AS 12 23 581 prinzipiell bekannt. Es handelt sich dabei um eine Einrichtung zur direkten Ophthalmoskopie, bei der die Netzhaut durch ein parallel auf das zu untersuchende Auge einfallendes Beobachtungsstrahlenbündel betrachtet wird, ohne die Erzeugung eines Zwischenbildes der Netzhaut, welches anschließend vergrößert wird. Die Netzhaut wird daher auch nicht mittels synchron bewegbarer spaltförmiger Blenden abgetastet und die Einstellung der Richtung des Beobachtungsstrahlenganges dient nicht zur Erhöhung des Kontrastes, sondern lediglich dazu, auch bei fehlsichtigen Augen die Gesichtsfelder von Beobachtung und Beleuchtung zusammenfallen zu lassen.

Weitere Vorteile und Einzelheiten der Erfindung werden im folgenden anhand der beiliegenden Zeichnung erläutert.

In dieser zeigt:
- Fig. 1: eine halbschematische Darstellung eines erfindungsgemäßen Gerätes zur Auflichtmikroskopie in Seitenansicht,
- Fig. 2: eine schematische Detailzeichnung eines Teiles der Beleuchtungseinrichtung in Vorderansicht,
- Fig. 3: die Verwendung eines erfindungsgemäßen Gerätes bei der indirekten Ophthalmoskopie,
- Fig. 4: ein als Durchlichtmikroskop ausgebildetes erfindungsgemäßes Gerät.

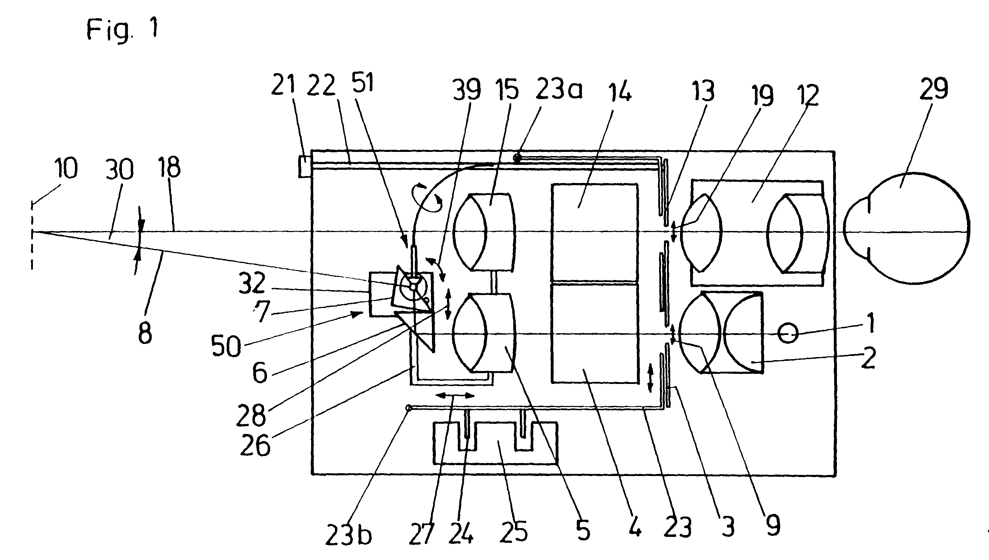

Das in Fig. 1 dargestellte Auflichtmikroskop weist eine Beleuchtungseinrichtung auf, welche eine Lichtquelle 1, einen Kondensor 2, eine spaltförmige Beleuchtungsblende 3, ein Umkehrprisma 4, ein Beleuchtungsobjektiv 5, ein Umlenkprisma 6 und ein als Beleuchtungsprisma 7 bezeichnetes weiteres Umlenkprisma beinhaltet. Von der Beleuchtungseinrichtung wird ein Beleuchtungsstrahlenbündel 8 erzeugt, das in Fig. 1 durch seinen zentralen Strahl gekennzeichnet ist. Das optische System bestehend aus Okular 12, spaltförmiger Beobachtungsblende 13, Umkehrprisma 14 und Beobachtungsobjektiv 15 legt ein Beobachtungsstrahlenbündel mit einem zentralen Strahl 18 fest. Die Ebene, die durch Beobachtungsstrahlenbündel 18 und Beleuchtungsstrahlenbündel 8 gebildet wird, steht dabei senkrecht zur Längsausdehnung der Spalte der Blenden 3,13. Die Beleuchtungsblende 3 und die Beobachtungsblende 13 sind jeweils in Zwischenbildebenen angeordnet und ihre fokusierten Spaltbilder sollen sich in der Objektebene 10 möglichst vollständig überlappen. Die Spaltbreiten der Blenden 3,13 sind, wie durch die Pfeile 9,19 angedeutet, verstellbar und zwar durch Verdrehung einer Rändelschraube 21, mit der über eine flexible Welle 22 ein in Fig. 1 nicht dargestellter Exzenter betätigt wird.

Um die Blenden 3,13 synchron zu bewegen und dadurch ihre Spaltbilder in der Objektebene 10 gleichzeitig um im wesentlichen die gleichen Auslenkungen zu verschieben, wodurch ein zu beobachtendes Objekt abgetastet werden kann, sind die Blenden 3,13 an einer federelastischen Trägervorrichtung 23 angeordnet, welche an ihren Enden 23a, 23b starr aufgehängt ist. Die Trägervorrichtung weist Tauchkerne 24 auf, welche mit einem mit Wechselstrom oder pulsierendem Gleichstrom betriebenen Elekromagneten 25 zur periodischen Auslenkung der federelastischen Trägervorrichtung 23 zusammen wirken. Über die Lage der Aufhängungspunkte 23a, 23b kann die effektive Länge der Trägervorrichtung 23 und damit ihre Eigenfrequenz verändert werden. Die Lage der Aufhängungspunkte 23a, 23b, der Ort der Anbringung der Tauchkeme 24 und die Frequenz des Wechselstromes werden dabei so aufeinander abgestimmt, daß vom Elektromagneten 25 eine Eigenfrequenz der Trägervorrichtung 23 resonant angeregt wird, sodaß eine ausreichend große laterale Auslenkung der Blenden 3,13 erzielt wird. Natürlich geht dabei die Federkonstante der Trägervorrichtung und ihre effektive Dämpfung ein. Die Größe der Auslenkung der Blenden 3,13, welche den beobachteten Bereich des Objektes bestimmt, kann u.a. über die Stärke der Anregung und die Größe der Dämpfung eingestellt werden. Die Frequenz, mit der die Schwingung der Trägervorrichtung 23 erfolgt, wird so gewählt, daß die Trägheit des Detektors, z.B. des menschlichen Auges 29, zu einem Verschmelzen der einzelnen Bilder führt. Beispielsweise eignet sich dazu eine Frequenz im Bereich von 50 Hz, die durch den mit Wechselstrom aus dem öffentlichen Netz betriebenen Elekromagneten 25 angeregt wird. Die Vorteile der beschriebenen Antriebseinrichtung für die Blenden 3,13 liegen insbesondere darin, daß die Antriebseinrichtung sehr platzsparend ausgebildet werden kann und nur zu einer äußerst geringen Lärmbelästigung führt.

Die Objektive 5,15 und die Prismen 6,7 sind gemeinsam an einem schematisch dargestellten Trägerrahmen 26 angebracht, der in Richtung des Pfeiles 27 längs der durch die Objektive 5,15 tretenden Strahlenbündel 8,18 verschiebbar ist. Dadurch kann die Objektweite, also der Abstand der Objektebene 10 von den Objektiven 5,15, bzw. die Vergrößerung und die Apertur eingestellt werden. Um zu gewährleisten, daß bei allen eingestellten Objektweiten die Spaltbilder der Beleuchtungsblende 3 und der Beobachtungsblende 13 in der Objektebene 10 zusammenfallen, ist eine weiter unten beschriebene Steuereinrichtung 50 vorgesehen, welche die Winkelstellung des Beleuchtungsprismas 7 und damit den Winkel 30 zwischen Beleuchtungsstrahlenbündel 8 und Beobachtungsstrahlenbündel 18 in Abhängigkeit von der Objektweite einstellt. Dabei ist normalerweise nicht störend, daß bei einer Veränderung des Winkels 30 aufgrund der Veränderung des optischen Weges des Beleuchtungsstrahlenbündels 8 das Spaltbild der Beleuchtungsblende 3 nicht mehr exakt in der Objektebene 10 des Beobachtungsstrahlenbündels 18 fokusiert ist. Sollte dies aber dennoch störend sein (besonders bei relativ großen Winkeln 30), könnte die Scheimpflugbedingung angewendet werden. Diese besagt, daß das Bild der Beleuchtungsblende 3 in der Objektebene 10 scharf ist, wenn sich die Objektebene 10, die "wirksame Objektivebene" und eine die Beleuchtungsblende 3 enthaltende Ebene in einer Linie schneiden. Mit "wirksamer Objektivebene" ist hier, da der Beleuchtungsstrahlengang 8 von den Umlenkprismen 6,7 geknickt wird, eine Ebene senkrecht zur Richtung des auf die Objektebene 10 treffenden Beleuchtungsstrahlenbündels 8 und durch das Objektiv 5 gemeint. Die Beleuchtungsblende 3 könnte somit zur scharf fokusierten Abbildung des Beleuchtungsspaltes in der Objektebene 10 entsprechend der Scheimpflugbedingung verkippt werden.

Weiters ist das Umlenkprisma 6 zusammen mit dem Beleuchtungsprisma 7 in die Richtung des Pfeiles 28, das heißt in eine Richtung senkrecht zur Richtung des Beobachtungsstrahlenbündels 18 verschiebbar. Der Normalabstand zwischen dem zentralen Strahl des Beobachtungsstrahlenbündsel 18 und dem Punkt des Beobachtungsprismas 7, an dem der zentrale Strahl des Beleuchtungsstrahlenbündels 8 umgelenkt wird, wird in der Folge als Prismenbasisabstand bezeichnet. Die bereits erwähnte Steuereinrichtung 50 stellt auch die Winkelstellung des Beleuchtungsprismas 7 und damit den Winkel 30 in Abhängigkeit vom Prismenbasisabstand derart ein, daß für jede Einstellung des Prismenbasisabstandes die Spaltbilder der Beobachtungsblende und der Beleuchtungsblende in der Objektebene 10 im wesentlichen zusammenfallen. Bei konstanten Spaltbreiten der Blenden 3,13 kann über den Prismenbasisabstand der gewünschte Kontrast der Abbildung gewählt werden. Andererseits kann auch vorgesehen sein, den Kontrast für verschiedene Prismenbasisabstände und für verschiedene Objektweiten konstant zu halten, indem eine Kopplungseinrichtung 51 zur Kopplung der Winkelstellung des Beleuchtungsprismas 7 und damit des Winkels 30 mit den Spaltbreiten der Blenden 3,13 vorgesehen ist.

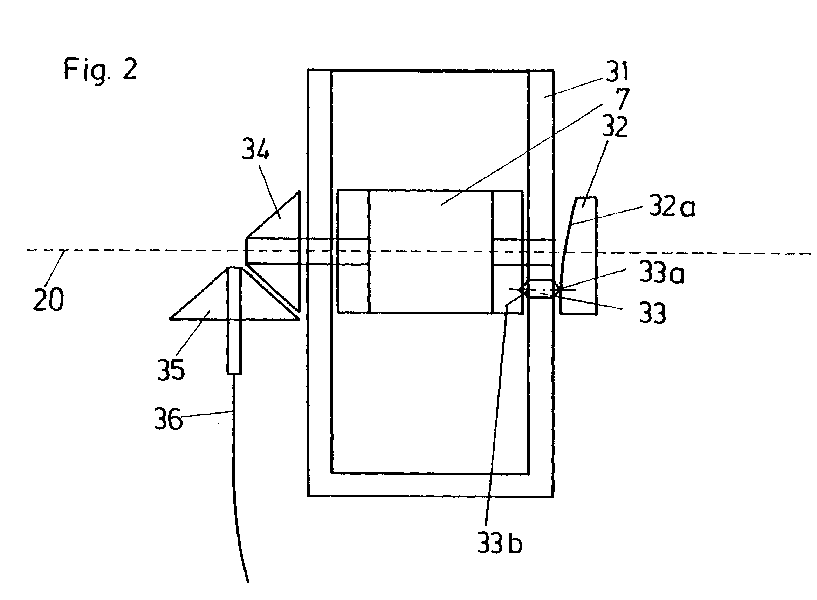

Die Kopplungseinrichtung 51 und die Steuereinrichtung 50 werden im folgenden anhand der Figuren 1 und 2 erläutert. Wie aus Fig. 2 ersichtlich ist, ist das Beleuchtungsprisma 7 drehbar an einem Rahmen 31 gelagert. Die Steuerplatte 32 ist in Fig. 1 in Draufsicht und in Fig. 2 in Seitenansicht sichtbar. Die Steuereinrichtung 50 zur Einstellung der Winkelstellung des Prismas 7 umfaßt eine Steuerplatte 32, deren Oberfläche als Steuerfläche 32a ausgebildet ist. An der Steuerfläche 32a liegt eine Spitze 33a eines Steuerstiftes 33 an. Im Bereich des anderen, ebenfalls spitz zulaufenden Endes 33b des Steuerstiftes 33 liegt dieser an einer Seitenfläche des Prismas 7 an, welches durch eine nicht dargestellte Feder unter einer Vorspannung steht und dadurch seitlich gegen den als Keil wirkenden Steuerstift 33 gedrückt wird. Bei einer Verstellung des Trägerrahmens 26 in Richtung des Pfeiles 27 oder bei einer Verstellung des Rahmens 31 relativ zum Trägerrahmen 26 in Richtung des Pfeiles 28 gleitet der Steuerstift 33 über die in drei Dimensionen entsprechend gekrümmte Steuerfläche 32a, wird dabei in eine Richtung senkrecht zur Seitenfläche des Prismas 7, an der er anliegt,verschoben und beeinflußt dadurch die Winkelstellung des Prismas 7. Der über die Steuerfläche 32a einzustellende Winkel 30 zwischen Beleuchtungsstrahlenbündel 8 und Beobachtungsstrahlenbündel 18 wird dabei vom Prismenbasisabstand Z und von der Objektweite O abgehängt und er ist gleich INVTAN (Z/O). Die Objektweite O hängt andererseits mit der Bildweite B, welche in Fig. 1 jeweils der Abstand zwischen dem Objektiv 5,15 und der zugehörigen Spaltblende 3,13 sein muß, über B=(O.F)/(O-F) zusammen, wenn F die Brennweite des Objektivs 5,15 bezeichnet. Für jede Stellung des Trägerrahmens 26 entlang des Pfeiles 27 und für jede Stellung des Rahmens 31 entlang des Pfeiles 28 ist somit der Winkel 30 bekannt und kann bei bekannter Geometrie des keilförmigen Endes 33b des Steuerstiftes 33 auf eine bestimmte Höhe der Steuerfläche 32 a abgebildet werden.

Die Koppeleinrichtung 51 zur Kopplung der Winkelstellung des Prismas 7 und der Spaltbreiten der Blenden 3,13 kann ein mit dem Prisma 7 verbundenes Kegelrad 34, ein mit diesem Kegelrad kämmendes Kegelrad 35 und eine biegsame Welle 36 zur Übertragung der Verdrehung des Kegelrades 35 aufweisen.

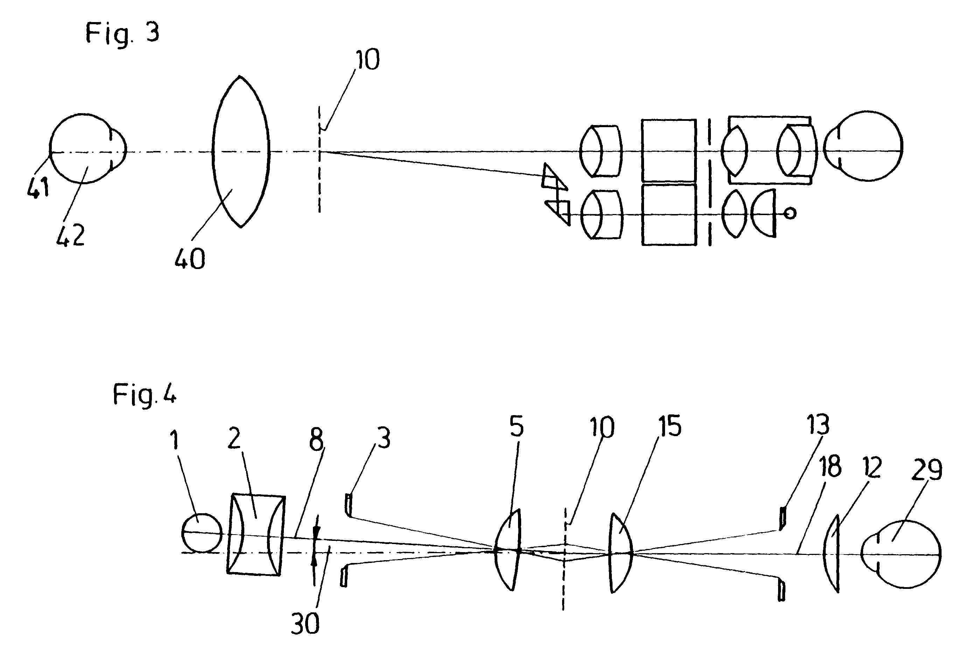

In Fig. 3 ist eine Anordnung zur Verwendung eines erfindungsgemäßen Gerätes bei der indirekten Ophthalmoskopie dargestellt. Mittels einer Ophthalmoskopielinse 40 wird die Netzhaut 41 eines Auges 42 reell als Zwischenbild abgebildet und zwar in der Objektebene 10 des erfindungsgemäßen Gerätes. Mit dem erfindungsgemäßen Gerät wird dieses Zwischenbild mit einer einstellbaren Vergrößerung vergrößert, wobei durch die schrägfokale Beleuchtung in Zusammenhang mit den synchron bewegten Blenden eine kontrastreiche Beobachtung eines entsprechenden Teiles der Netzhaut möglich ist. Mit zunehmender Vergrößerung vergrößert sich auch die Apertur und damit die Auflösung. Andererseits kann durch die Wahl der Vergrößerung die gewünschte Tiefenschärfe eingestellt werden.

Eine Ausführungsform eines erfindungsgemäßen Gerätes als Durchlichtmikroskop ist in Fig. 4 dargestellt. Das Beleuchtungsstrahlenbündel 8 und das Beobachtungsstrahlenbündel 18 treffen hier von verschiedenen Seiten auf die Objektebene 10 auf. Analoge Teile der Einrichtung sind mit den gleichen Bezugszeichen wie in Fig. 1 versehen. Der Neigungswinkel 30, der in diesem Fall zwischen dem Beleuchtungsstrahlenbündel 8 und der Verlängerung des Beobachtungsstrahlenbündels 18 gemessen wird, wird entsprechend der gewünschten Abschwächung des Nullbeugungslichtes eingestellt, wodurch Kontrast und Blendfreiheit erhöht werden. Eine Veränderung des Winkels 30 kann bei der Ausbildung als Durchlichtmikroskop einfach dadurch erfolgen, daß die Beleuchtungseinrichtung auf einem in Fig. 4 nicht dargestellten Träger angeordnet ist, der um eine Achse senkrecht zur Bildebene und durch den Schnittpunkt der Objektebene 10 mit dem Beobachtungsstrahlenbündel 18 und dem Beleuchtungsstrahlenbündel 8 verschwenkbar ist. Bei einer Veränderung der Objektweite durch Verstellung des Objektives 15 ist bei diesem Ausführungsbeispiel keine Steuerungseinrichtung notwendig, welche den Winkel 30 verstellt. Es könnte aber eine Kopplungseinrichtung zwischen dem Winkel 30 und den Spaltbreiten der Blenden 3,13 vorgesehen sein.

Anstelle einer Beobachtung mit dem Auge 29 kann auch eine mit dem Gerät gekoppelte Foto- oder Video-Kamera verwendet werden. Laserlicht kann für therapeutische Zwecke eingekoppelt werden.

Anstelle von elektromagnetischer Strahlung im sichtbaren Bereich kann auch elektromagnetische Strahlung in einem anderen Frequenzbereich verwendet werden, beispielsweise Mikrowellenstrahlung. Zur Detektion kann dann natürlich nicht das menschliche Auge verwendet werden, sondern ein für die verwendete Strahlung geeigneter Detektor. Weiters ist es auch denkbar und möglich, eine andere Art der Strahlung, welche Welleneigenschaften aufweist, zu verwenden, wie beispielsweise Elektronenstrahlen oder auch Schallwellen.

## Patentansprüche

1. Abbildendes optisches Gerät mit
einem ein Beobachtungsstrahlenbündel (18) festlegenden optischen System,
einer ein Beleuchtungsstrahlenbündel (8) erzeugenden Beleuchtungseinrichtung, wobei das Beobachtungsstrahlenbündel (18) und das Beleuchtungsstrahlenbündel (8) schräg zueinander auf die Objektebene (10) treffen,
einer in einer Zwischenbildebene des Beobachtungsstrahlenbündels (18) sich bewegenden spaltförmigen Beobachtungsblende (13) und
einer in einer Zwischenbildebene des Beleuchtungsstrahlenbündels (8) synchron sich bewegenden spaltförmigen Beleuchtungsblende (3), wobei die Spaltbilder der Beobachtungsblende (13) und der Beleuchtungsblende (3) in der Objektebene (10) im wesentlichen zusammenfallen und die Ebene, die durch Beobachtungsstrahlenbündel (18) und Beleuchtungsstrahlenbündel (8) gebildet wird, senkrecht zur Längsausdehnung der Spalte der Beobachtungsblende (13) und der Beleuchtungsblende (3) liegt.

2. Optisches Gerät nach Anspruch 1, **dadurch gekennzeichnet, daß** der Neigungswinkel (30) zwischen dem auf die Objektebene (10) treffenden Beobachtungsstrahlenbündel (18) und dem auf die Objektebene (10) treffenden Beleuchtungsstrahlenbündel (8) zwischen 0,5° und 30° liegt.

3. Optisches Gerät nach Anspruch 2, **dadurch gekennzeichnet, daß** der Neigungswinkel (30) zwischen Beobachtungsstrahlenbündel (18) und Beleuchtungsstrahlenbündel (8) einstellbar ist.

4. Optisches Gerät nach Anspruch 3, **dadurch gekennzeichnet, daß** bei der Ausgestaltung als Auflichtmikroskop zur Einstellung des Neigungswinkels (30) zwischen Beobachtungsstrahlenbündel (18) und Beleuchtungsstrahlenbündel (8) ein in eine Verschiebungsrichtung (28) senkrecht zur Richtung des Beobachtungsstrahlenbündels (18) verschiebbares und um eine Achse (20) senkrecht zur Ebene, die durch das Beobachtungsstrahlenbündel (18) und das Beleuchtungsstrahlenbündel (8) definiert ist, verdrehbares Beleuchtungsprisma (7) im Strahlengang des Beleuchtungsstrahlenbündels (8) vorgesehen ist.

5. Optisches Gerät nach Anspruch 4, **gekennzeichnet durch** eine Steuereinrichtung (50) zur Verdrehung des Beleuchtungsprismas (7) um seine Achse (20) in Abhängigkeit von der Position des Beleuchtungsprismas (7) entlang der Verschiebungsrichtung (28) derart, daß die Spaltbilder der Beobachtungsblende (13) und der Beleuchtungsblende (3) bei allen Positionen des Prismas entlang der Verschiebungsrichtung (28) in der Objektebene (10) im wesentlichen zusammenfallen.

6. Optisches Gerät nach einem der Ansprüche 3 bis 5, **dadurch gekennzeichnet, daß** die Spaltbreiten der Beobachtungsblende (13) und der Beleuchtungsblende (3) einstellbar sind und daß eine Kopplungseinrichtung (51) zur Kopplung der Einstellung des Neigungswinkels (30) zwischen Beobachtungsstrahlenbündel (18) und Beleuchtungsstrahlenbündel (8) mit der Einstellung der Spaltbreiten vorhanden ist.

7. Optisches Gerät nach Anspruch 6, **dadurch gekennzeichnet, daß** die Kopplungseinrichtung (51) die Spaltbreiten mit der Winkelstellung des Beleuchtungsprismas um seine Drehachse (20) koppelt.

8. Optisches Gerät nach einem der Ansprüche 4 bis 7, **dadurch gekennzeichnet, daß** bei der Ausgestaltung als Auflichtmikroskop die Objektweiten von Beobachtungsstrahlenbündel (18) und Beleuchtungsstrahlenbündel (8) synchron einstellbar sind, wobei der Neigungswinkel (30) zwischen Beobachtungsstrahlenbündel (18) und Beleuchtungsstrahlenbündel (8) von einer Steuereinrichtung (50) derart eingestellt wird, daß die Spaltbilder der Beobachtungsblende (13) und der Beleuchtungsblende (3) bei den verschiedenen Objektweiten jeweils in der Objektebene (10) zusammenfallen.

9. Optisches Gerät nach Anspruch 8, **dadurch gekennzeichnet, daß** die Steuereinrichtung (50) den Neigungswinkel (30) durch Verdrehung des Beleuchtungsprismas (7) einstellt.

10. Optisches Gerät nach einem der Ansprüche 5,8 oder 9, **dadurch gekennzeichnet, daß** die Steuereinrichtung (50) eine gekrümmte Steuerfläche (32a) aufweist.

11. Optisches Gerät nach Anspruch 10, **dadurch gekennzeichnet, daß** die Steuereinrichtung (50) einen Stift (33) zur Verdrehung des Beleuchtungsprismas (7) aufweist, dessen eines Ende (33b) mit dem Beleuchtungsprisma (7) in Verbindung steht und dessen anderes Ende (33a) an der Steuerfläche (32a) anliegt und bei einer Verschiebung des Beleuchtungsprismas (7) entlang der Steuerfläche (32a) verschoben wird.

12. Optisches Gerät nach Anspruch 11, **dadurch gekennzeichnet, daß** das mit dem Beleuchtungsprisma (7) in Verbindung stehende Ende (33b) des Steuerstiftes (33) keilförmig ausgebildet ist und an einer Seitenfläche des unter Vorspannung gegen den Steuerstift (33) gedrückten Beleuchtungsprismas (7) anliegt.

13. Optisches Gerät nach einem der Ansprüche 1 bis 12, **dadurch gekennzeichnet, daß** im Strahlengang des Beobachtungsstrahlenbündels (18) und im Strahlengang des Beleuchtungsstrahlenbündels (8) Umkehrprismen (4,14) vorgesehen sind.

## Claims

1. Optical imaging device with
an optical system establishing an observation beam (18),
a lighting apparatus producing a lighting beam (8), wherein the observation beam (18) and the lighting beam (8) are incident on the object plane (10)
at an angle to one another,
a slit-shaped observation diaphragm moving in an intermediate image plane of the observation beam (18), and
a slit-shaped lighting diaphragm moving synchronously in an intermediate image plane of the lighting beam, wherein the slit images of the observation diaphragm (13) and of the lighting diaphragm (3) substantially coincide, and the plane formed by the observation beam (18) and the lighting beam (8) is perpendicular to the longitudinal extent of the slits in the observation diaphragm (13) and the lighting diaphragm (3).

2. Optical device according to claim 1, **characterised in that** the angle of inclination (30) between the observation beam (18) incident on the object plane (10) and the lighting beam (8) incident on the object plane (10) is between 0,5° and 30°.

3. Optical device according to claim 2, **characterised in that** the angle of inclination (30) between the observation beam (18) and the lighting beam (8) is adjustable.

4. Optical device according to claim 3, **characterised in that** the case of configuration as a reflected light microscope, in the beam path of the lighting beam (8) a lighting prism (7) is provided moveable in a direction of movement (28) perpendicular to the direction of the observation beam (18) and rotatable about an axis perpendicular to said plane which is defined by the observation beam (18) and the lighting beam (8).

5. Optical device according to claim 4, **characterised by** a control apparatus (50) for rotating the lighting prism (7) about an axis (20) dependent upon the position of the lighting prism (7) along the direction of movement (28) such that the slit images of the observation diaphragm (13) and of the lighting diaphragm (3) substantially coincide in the object plane (10) at all positions of the prism along the direction of movement (28).

6. Optical device according to one of claims 3 to 5, **characterised in that** the slit widths of the observation diaphragm (13) and of the lighting diaphragm (3) are adjustable and that a coupling apparatus (51) for coupling the adjustment of the angle of inclination (30) between the observation beam (18) and the lighting beam (8), with adjustment of the slit widths, is present.

7. Optical device according to claim 6, **characterised in that** the coupling apparatus (51) couples the slit widths to the angle of the lighting prism about its axis of rotation (20).

8. Optical device according to one of claims 4 to 7, **characterised in that** the case of configuration as a transmitted light microscope, the object distances from the observation beam (18) and the lighting beam (8) can be adjusted synchronously, wherein the angle of inclination (30) between the observation beam (18) and lighting beam (8) are adjusted by means of a control apparatus (50) such that the slit images of the observation diaphragm (13) and of the lighting diaphragm (3) always coincide in the object plane at the different object distances.

9. Optical device according to claim 8, **characterised in that** the control apparatus (50) adjusts the angle of inclination (30) by rotation of the lighting prism (7).

10. Optical device according to one of claims 5, 8 or 9, **characterised in that** the control apparatus (50) is provided with a curved control surface (32a).

11. Optical device according to claim 10, **characterised in that** the control apparatus (50) is provided with a pin (33) for rotating the lighting prism (7), one end of which (33b) is connected to the lighting prism (7), and the other end (33a) of which lies against the control surface (32a), and is moved when the lighting prism (7) is moved along the control surface (32a).

12. Optical device according to claim 11, **characterised in that** the end (33b) of the control pin (33) in contact with the lighting prism (7) is configured as a wedge and lies against a lateral surface of the lighting prism (7) which is pressed under tension against the control pin (33).

13. Optical device according to one of claims 1 to 12, **characterised in that** deviating prisms (4,14) are provided in the beam path of the observation beam (18) and in the beam path of the lighting beam (8).

## Revendications

1. Appareil d'imagerie optique comprenant
- un système optique définissant un faisceau lumineux d'observation (18)
- une installation produisant un faisceau lumineux d'éclairage (8), dans lequel le faisceau lumineux d'observation (18) et le faisceau lumineux d'éclairage (8) se rencontrent sur le plan de l'objet (10),
- un écran d'observation (13) comportant une fente, mobile dans un plan focal intermédiaire du faisceau lumineux d'observation (18) et
- un écran d'éclairage comportant une fente (3), se déplaçant de manière synchrone dans un plan focal intermédiaire du faisceau lumineux d'éclairage (8), dans lequel les images des fentes des écrans d'observation (13) et d'éclairage (3) coïncident en grande partie sur le plan de l'objet (10) et le plan formé par le faisceau lumineux d'observation (18) et le faisceau d'éclairage (8) est perpendiculaire à la longueur des fentes de l'écran d'observation (13) et de l'écran d'éclairage (3).

2. Appareil optique selon la revendication 1, **caractérisé en ce que** l'angle d'inclinaison (30) entre le faisceau d'observation (18) rencontrant le plan de l'objet (10) et le faisceau lumineux d'éclairage (8) rencontrant le plan de l'objet (10) est compris entre 0,5° et 30°.

3. Appareil optique selon la revendication 2, **caractérisé en ce que** l'angle d'inclinaison (30) entre le faisceau lumineux d'observation (18) et le faisceau lumineux d'éclairage (8) est ajustable.

4. Appareil optique selon la revendication 3, **caractérisé en ce que**, lors d'une utilisation en tant que microscope à éclairage incident pour ajuster l'angle d'inclinaison (30) entre le faisceau lumineux d'observation (18) et le faisceau lumineux d'éclairage (8), un prisme d'éclairage déplaçable (7), mobile dans une direction de mouvement (28) perpendiculaire à la direction du faisceau lumineux d'observation (18), et autour d'un axe (20) perpendiculaire au plan défini par le faisceau lumineux d'observation (18) et le faisceau lumineux d'éclairage (8), est prévu sur la marche des rayons du faisceau lumineux d'éclairage (8).

5. Appareil optique selon la revendication 4, **caractérisé par** un dispositif de réglage (50) pour faire tourner le prisme d'éclairage (7) autour de son axe (20) en fonction de la position du prisme d'éclairage (7) le long de la direction de mouvement (28) tel que les images des fentes de l'écran d'observation (13) et de l'écran d'observation (3) coïncident quasiment, pour toutes les positions du prisme le long de la direction de mouvement, au niveau du plan de l'objet.

6. Appareil optique selon l'une quelconque des revendications 3 à 5, **caractérisé en ce que** les largeurs des fentes de l'écran d'observation (13) et de l'écran d'éclairage (3) sont ajustables, et **en ce qu'**un dispositif de couplage (51) est prévu pour ajuster le réglage de l'angle d'inclinaison (30) entre le faisceau lumineux d'observation (18) et le faisceau lumineux d'éclairage (8) en fonction de la largeur des fentes.

7. Appareil optique selon la revendication 6, **caractérisé en ce que** le dispositif de couplage (15) couple les largeurs des fentes avec la position angulaire du prisme d'éclairage par rapport à son axe de rotation (20)

8. Appareil optique selon l'une quelconque des revendications 4 à 7, **caractérisé en ce que**, lors d'une utilisation en tant que microscope à éclairage incident, les largeurs objet du faisceau lumineux d'observation (18) et du faisceau lumineux d'éclairage (8) sont ajustables de manière synchrone, dans lequel l'angle d'inclinaison (30) entre le faisceau lumineux d'observation (18) et le faisceau lumineux d'éclairage (8) est ajusté par un dispositif de réglage (50) de sorte que les images des fentes de l'écran d'observation (13) et de l'écran d'éclairage (3) coïncident, pour des largeurs d'objet différentes, sur le même plan d'objet.

9. Appareil optique selon la revendication 8, **caractérisé en ce que** le dispositif de réglage (50) règle l'angle d'inclinaison (30) par rotation du prisme d'éclairage (7).

10. Appareil optique selon l'une quelconque des revendications 5, 8 ou 9, **caractérisé en ce que** le dispositif de réglage (50) présente une surface de réglage courbe (32a).

11. Appareil optique selon la revendication 10, **caractérisé en ce que** le dispositif de réglage (50) présente un pion (33) pour faire tourner le prisme d'éclairage (7), dont une extrémité (33b) est couplée au prisme de lumière (7) et l'autre extrémité (33a) est adjacente à la surface de réglage (32a) et est mobile le long de la surface de réglage (32a), lorsque l'on modifie la position du prisme d'éclairage (7).

12. Appareil optique selon la revendication 11, **caractérisé en ce que** l'extrémité couplée au prisme d'éclairage (7) du pion de réglage (33) est cunéiforme et adjacente à une face latérale du prisme d'éclairage (7) pressé sous tension contre le pion de réglage (33).

13. Appareil optique selon l'une quelconque des revendications 1 à 12, **caractérisé en ce que** l'on a prévu des prismes redresseurs (4,14) sur la marche des rayons du faisceau lumineux d'observation (18) et sur la marche des rayons du faisceau lumineux d'éclairage (8).
